(19) Europäisches Patentamt
European Patent Office
Office européen des brevets

(11) Veröffentlichungsnummer: **0 002 180**
**B1**

(12) # EUROPÄISCHE PATENTSCHRIFT

(45) Veröffentlichungstag der Patentschrift: **12.08.81**

(51) Int. Cl.³: **C 07 D 231/14,** A 01 N 43/56, C 07 D 401/04, C 07 D 403/04, C 07 D 413/04, C 07 D 417/04

(21) Anmeldenummer: **78101074.9**

(22) Anmeldetag: **05.10.78**

(54) **Substituierte 3-Aminopyrazole, diese enthaltende Herbizide und Verfahren zur Bekämpfung unerwünschten Pflanzenwuchses.**

(30) Priorität: **22.10.77 DE 2747531**

(43) Veröffentlichungstag der Anmeldung: **13.06.79 Patentblatt 79/12**

(45) Bekanntmachung des Hinweises auf die Patenterteilung: **12.08.81 Patentblatt 81/32**

(84) Benannte Vertragsstaaten: **BE CH DE FR GB LU NL SE**

(56) Entgegenhaltungen:
**DE-A-2 260 485**
**DE-A-2 404 795**
**DE-A-2 513 750**
**DE-A-2 701 091**
**FR-A-2 262 663**

(73) Patentinhaber: **BASF Aktiengesellschaft, Carl-Bosch-Strasse 38, D-6700 Ludwigshafen (DE)**

(72) Erfinder: **Plath, Peter, Dr., Berner Weg 24, D-6700 Ludwigshafen (DE)**
Erfinder: **Wuerzer, Bruno, Dr.,Dipl.-Landwirt, Wilhelm-Busch-Strasse 55, D-6703 Limburgerhof (DE)**
Erfinder: **Rohr, Wolfgang, Dr., Gontardstrasse 4, D-6800 Mannheim 1 (DE)**

**EP 0 002 180 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

phenyl, 2,5-Dichlorphenyl, 2-Methyl-3-chlorphenyl, 2-Methyl-5-chlorphenyl, 2-Methoxy-5-chlorphenyl, 3-Tetrafluoräthoxy-phenyl, 2-Chlor-5-trifluor-methylphenyl, 2-Methoxy-5-methylphenyl.

$R^4$ kann beispielsweise folgende Reste bedeuten:

Methoxycarbonyl, Isopropoxycarbonyl, Acetyl.

Wenn $R^2$ und $R^3$ zusammen einen Polymethylenrest bilden, kann dieser gegebenenfalls ein Sauerstoffatom enthalten. Zusammen mit dem in 3-Stellung des Pyrazols stehenden Stickstoffatomen resultieren dann z. B. folgende Ringe:

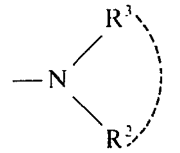
= Tetramethylenimino, Pentamethylenimino, Hexamethylenimino, Morpholin.

Verfahren zur Herstellung der neuen Pyrazolderivate sind z. B. in Chem. Ber. 98, 259 (1965) beschrieben.

Ein anderes Verfahren besteht in der Kondensation von Aldehyden, Ketonen oder $\alpha$-Ketocarbonsäurederivaten mit 3-Amino-4-alkoxycarbonylpyrazolen, die gegebenenfalls in 5-Stellung substituiert sein können, und anschließender Hydrierung der so erhaltenen Schiffschen Basen.

Die in 1- oder 2-Stellung acylierten Pyrazole werden durch nachträgliche Acylierung der 3-Aminopyrazolderivate nach bekannten Verfahren erhalten.

Die Herstellung der Salze der neuen Pyrazole erfolgt durch Umsetzung der Pyrazole mit den Säuren in einem Lösungsmittel. Die Herstellung der Metallkomplexverbindungen der neuen Pyrazole erfolgt durch Umsetzung der Pyrazole mit den Metallsalzen in einem Lösungsmittel.

Die zur Herstellung der neuen Pyrazole gewählten Verfahren werden durch die folgenden Beispiele erläutert.

## Beispiel 1

### 3-Anilino-4-isopropoxycarbonyl-5-methylpyrazol

Zu einer Suspension von 33,5 g 4-Phenylthiosemicarbazid in 150 ml Tetrahydrofuran gibt man 35,7 g 2-Chloracetessigsäure-isopropylester. Nach 30 min Rühren wird zum Sieden erhitzt und nach weiteren 30 min abkühlen gelassen. Man isoliert den ausgefallenen Feststoff durch Absaugen, wäscht mit Aceton nach und erhitzt den verbleibenden Rückstand in einem Wasser/Aceton-Gemisch (1 : 1). Nach Abgießen vom abgeschiedenen Schwefel läßt man abkühlen, neutralisiert mit wäßriger $NaHCO_3$-Lösung und isoliert den ausfallenden Feststoff.

Fp. 138 – 139° C (aus Toluol).

## Beispiel 2

### 3-Isobutylamino-4-methoxycarbonyl-5-methylpyrazol

Zu einer Suspension von 14,7 g 4-Isobutyl-thiosemicarbazid in 150 ml Tetrahydrofuran gibt man 15,1 g 2-Chloracetessigsäuremethylester. Nach 30 min Rühren wird zum Sieden erhitzt. Man läßt nach 1 Stunde Nachrühren bei 65° C abkühlen, trennt den ausgefallenen Feststoff ab und wäscht mit Aceton nach. Der verbleibende Rückstand wird in Wasser heiß gelöst, vom ausgefallenen Schwefel abfiltriert und nach dem Abkühlen mit wäßriger Ammoniak-Lösung vermischt. Man isoliert nach Absaugen und Trocknen das Produkt 3-Isobutylamino-4-methoxycarbonyl-5-methylpyrazol.

Fp. 100 – 103° C.

## Beispiel 3

### 3-Piperidino-4-methoxycarbonyl-5-methylpyrazol

Es wurde nach der in J. pr. Ch. (2) 159, 189 (1941) angegebenen Methode 4,4-Pentamethylenthiosemicarbazid (Fp. 98 – 99° C) hergestellt.

32 g des 4,4-Pentamethylenthiosemicarbazids werden in 100 ml Tetrahydrofuran (THF) gegeben und mit 31 g 2-Chloracetessigsäuremethylester versetzt. Nach 1 Stunde Nachrühren bei Raumtemperatur wird der entstandene Feststoff abgesaugt, mit Aceton nachgewaschen, in $H_2O$ gelöst und mit $NH_3$-Wasser bis zur alkalischen Reaktion (pH 8) versetzt. Das abgeschiedene Öl wird durch Extraktion mit $CH_2Cl_2$ vom Schwefel abgetrennt. Nach dem Einengen wird aus Aceton umkristallisiert.

Fp. 101 – 102° C.

Fortsetzung

| Nr. | $R^2$ | $R^3$ | $R^5$ | $R^6$ | Fp. (°C) |
|---|---|---|---|---|---|
| 37 | H | 3,4-Dichlorphenyl | Methyl | $OCH_3$ | 234–236 |
| 38 | H | 2,5-Dichlorphenyl | Methyl | $OCH_3$ | 251–253 |
| 39 | H | 3,5-Dichlorphenyl | Methyl | $OCH_3$ | |
| 40 | H | 3-Chlor-4-fluorphenyl | Methyl | $OCH_3$ | 212 |
| 41 | H | 3,4-Difluorphenyl | Methyl | $OCH_3$ | 204 |
| 42 | H | 2-Methyl-3-chlorphenyl | Methyl | $OCH_3$ | 224–226 |
| 43 | H | 2-Methyl-4-chlorphenyl | Methyl | $OCH_3$ | 205–206 |
| 44 | H | 2-Methyl-5-chlorphenyl | Methyl | $OCH_3$ | mehr als 240 |
| 55 | H | Methyl | Methyl | $OCH_3$ | 155–157 |
| 56 | H | Äthyl | Methyl | $OCH_3$ | 143–144 |
| 57 | H | n-Propyl | Methyl | $OCH_3$ | |
| 58 | H | Allyl | Methyl | $OCH_3$ | 128–130 |
| 59 | H | Isopropyl | Methyl | $OCH_3$ | 100–103 |
| 60 | H | 2-Methylpropen-1-yl-3 | Methyl | $OCH_3$ | |
| 61 | H | 3-Methylpropen-1-yl-3 | Methyl | $OCH_3$ | |
| 65 | H | 3,3-Dimethylpropen-1-yl-3 | Methyl | $OCH_3$ | |
| 66 | H | n-Butyl | Methyl | $OCH_3$ | 117–118 |
| 67 | H | iso-Butyl | Methyl | $OCH_3$ | 100–103 |
| 68 | H | sek.-Butyl | Methyl | $OCH_3$ | 81–83 |
| 69 | H | tert.-Butyl | Methyl | $OCH_3$ | 115 |
| 70 | H | Pentyl-2 | Methyl | $OCH_3$ | Öl |
| 71 | H | Pentyl-2 | Methyl | $OCH_3$ | 81–82 |
| 72 | H | 1-Methoxypropyl-2 | Methyl | $OCH_3$ | $n_D^{21}$ = 1,5122 |
| 73 | H | 1,1-Dimethoxypropyl-2 | Methyl | $OCH_3$ | |
| 77 | H | 1-Methoxybutyl-2 | Methyl | $OCH_3$ | Öl |
| 78 | H | 3-Methoxypropyl-1 | Methyl | $OCH_3$ | 107–108 |
| 79 | H | 1,3-Dimethoxypropyl-2 | Methyl | $OCH_3$ | |
| 80 | H | Cyclopentyl | Methyl | $OCH_3$ | 83–85 |
| 82 | H | Cyclohexyl | Methyl | $OCH_3$ | 90–92 |
| 87 | H | Benzyl | Methyl | $OCH_3$ | 160–161 |
| 88 | H | 2-Phenyläthyl | Methyl | $OCH_3$ | 181–182 |

## Beispiel 5

### 1-Acetyl-3-anilino-4-methoxycarbonyl-5-methylpyrazol

Man löst 23 g 3-Anilino-4-methoxycarbonyl-5-methylpyrazol in 51 g Essigsäureanhydrid und erhitzt zum Sieden. Nach 15 min Sieden läßt man abkühlen, gießt auf Eiswasser und läßt 30 min rühren. Der abgeschiedene Feststoff wird isoliert und aus Methanol umkristallisiert.

Fp. 115 – 116° C.

Die Stellung der Acetylgruppe wurde anhand des IR-Spektrums erkannt (CO-Bande bei 1720 $cm^{-1}$). Dieser Befund schließt nur aus, daß der Anilinorest acetyliert wurde. Durch Röntgenstrukturanalyse ließ sich zeigen, daß der Actylrest in Position 1 des Pyrazolrings sitzt.

## Beispiel 6

### 1-Acetyl-3-cyclohexylamino-4-methoxycarbonyl-5-methylpyrazol

Herstellung erfolgt analog Beispiel 5. Fp. 140 – 142° C.

Weitere Derivate von Pyrazolen, die nach einer der Methoden der Beispiele oder anderen bekannten Methoden erhalten wurden, sind in der folgenden Aufzählung zusammengestellt.

| Nr. | $R^1$ | $R^2$ | $R^3$ | $R^4$ | $R^5$ | Fp. (°C) |
|---|---|---|---|---|---|---|
| 141 | Formyl | Wasserstoff | Phenyl | $-CO_2-CH_3$ | Methyl | 114–115 |
| 142 | Formyl | Wasserstoff | sek.-Butyl | $-CO_2-CH_3$ | Methyl | |
| 143 | Formyl | Wasserstoff | tert.-Butyl | $-CO_2-CH_3$ | Methyl | |
| 144 | Acetyl | Wasserstoff | Phenyl | $-CO_2-CH_3$ | Methyl | 116 |
| 145 | Chloracetyl | Wasserstoff | Phenyl | $-CO_2-CH_3$ | Methyl | 145–147 |
| 148 | Trifluoracetyl | Wasserstoff | Phenyl | $-CO_2-CH_3$ | Methyl | |
| 149 | Propionyl | Wasserstoff | Phenyl | $-CO_2-CH_3$ | Methyl | 120–122 |
| 151 | Methoxycarbonyl | Wasserstoff | Phenyl | $-CO_2-CH_3$ | Methyl | 142 |
| 152 | Äthoxycarbonyl | Wasserstoff | Phenyl | $-CO_2-CH_3$ | Methyl | 130–132 |
| 153 | Acetyl | Wasserstoff | 2-Methylphenyl | $-CO_2-CH_3$ | Methyl | 145–147 |
| 154 | Acetyl | Wasserstoff | 3-Methylphenyl | $-CO_2-CH_3$ | Methyl | 86–89 |
| 155 | Acetyl | Wasserstoff | 2-Fluorphenyl | $-CO_2-CH_3$ | Methyl | 144–146 |
| 156 | Acetyl | Wasserstoff | 3-Fluorphenyl | $-CO_2-CH_3$ | Methyl | 135–136 |
| 157 | Acetyl | Wasserstoff | 4-Fluorphenyl | $-CO_2-CH_3$ | Methyl | 132–134 |
| 158 | Acetyl | Wasserstoff | sek.-Butyl | $-CO_2-CH_3$ | Methyl | 76–77 |
| 159 | Acetyl | Wasserstoff | tert.-Butyl | $-CO_2-CH_3$ | Methyl | 120 |
| 160 | Acetyl | Wasserstoff | Cyclopentyl | $-CO_2-CH_3$ | Methyl | 96–98 |
| 161 | Acetyl | Wasserstoff | Cyclohexyl | $-CO_2-CH_3$ | Methyl | 140–142 |
| 162 | Acetyl | Wasserstoff | Norbonyl | $-CO_2-CH_3$ | Methyl | 139–142 |
| 164 | Acetyl | Methyl | Cyclohexyl | $-CO_2-CH_3$ | Methyl | 80–82 |
| 165 | Acetyl | Methyl | Phenyl | $-CO_2-CH_3$ | Methyl | 88–90 |

Fortsetzung

| Nr | $R^1$ | $R^2$ | $R^3$ | $R^4$ | $R^5$ | Fp (°C) |
|---|---|---|---|---|---|---|
| 204 | H | H | 3-Fluorphenyl | $-CO_2CH_3$ | H | 160 |
| 205 | H | H | 2-Fluorphenyl | $-CO_2CH_3$ | H | |
| 206 | H | H | 3-Methoxyphenyl | $-CO_2CH_3$ | H | |
| 212 | H | H | 2,3-Dichlor-phenyl | $-CO_2CH_3$ | $CH_3$ | 253–255 |
| 213 | H | H | 2,3-Dimethylphenyl | $-CO_2CH_3$ | $CH_3$ | 216–218 |
| 217 | H | H | 2-Chlor-5-trifluor-methylphenyl | $-CO_2CH_3$ | $CH_3$ | mehr als 240 |
| 218 | H | H | 2,5-Dimethoxyphenyl | $-CO_2CH_3$ | $CH_3$ | 180–181 |
| 219 | H | H | 2-Methoxy-5-methylphenyl | $-CO_2CH_3$ | $CH_3$ | 192 |
| 220 | Acetyl | H | sec.-Butyl | $COOCH_3$ | H | 76–77 |
| 223 | Acetyl | H | Phenyl | $-CO_2C_3H_7$-t | $-CH_3$ | 121 |
| 227 | H | H | 3-Methyl-4-fluor-phenyl | $-CO_2CO_3$ | $-CH_3$ | 192–194 |
| 228 | Acetyl | H | 3-Methyl-4-fluor-phenyl | $-CO_2CH_3$ | $-CH_3$ | 115 |
| 229 | Acetyl | H | 2-Methyl-5-fluor-phenyl | $-CO_2CH_3$ | $-CH_3$ | 163–164 |
| 230 | H | H | 2-Methyl-5-fluor-phenyl | $-CO_2CH_3$ | $-CH_3$ | 230–232 |
| 231 | Acetyl | H | 2-Methyl-4-chlor-phenyl | $-CO_2CH_3$ | $-CH_3$ | 191–192 |
| 232 | Acetyl | H | 3,4-Dichlorphenyl | $-CO_2CH_3$ | $-CH_3$ | 142–143 |
| 233 | Acetyl | H | 4-Methylphenyl | $-CO_2CH_3$ | $-CH_3$ | 130–132 |
| 234 | Acetyl | H | 4-Chlorphenyl | $-CO_2CH_3$ | $-CH_3$ | 150–153 |
| 235 | Acetyl | H | -Phenyläthyl | $-CO_2CH_3$ | $-CH_3$ | 92–93 |
| 236 | Acetyl | H | 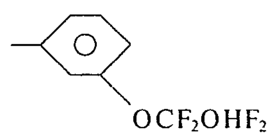 | $-CO_2CH_3$ | $-CH_3$ | 135–136 |
| 237 | Acetyl | H | 2,5-Dimethoxyphenyl | $-CO_2CH_3$ | $-CH_3$ | 177–178 |
| 238 | Acetyl | H | 2,5-Methoxy-5-chlorphenyl | $-CO_2CH_3$ | $-CH_3$ | 221–223 |
| 239 | Acetyl | H | 2,3-dichlorphenyl | $-CO_2CH_3$ | $-CH_3$ | 124–125 |
| 240 | Acetyl | H | 2,5-Dichlorphenyl | $-CO_2CH_3$ | $-CH_3$ | 180–182 |
| 241 | Acetyl | H | 2-Trifluormethyl-phenyl | $-CO_2CH_3$ | $-CH_3$ | 147–148 |

äthoxyliertes Isooctylphenyl-, Octylphenol-, Nonylphenol, Alkylphenolpolyglykoläther, Tributylphenylpolyglykoläther, Alkalarylpolyätheralkohle, Isotridecylalkohol, Fettalkoholäthylenoxid-Kondensate, äthoxyliertes Rizinusöl, Polyoxyäthylenalkyläther, äthoxyliertes Polyoxypropylen, Laurylalkoholpolyglykolätheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsilfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Die Formulierungen enthalten zwischen 0,1 und 95 Gewichtsprozent Wirkstoff, vorzugsweise zwischen 0,5 und 90 Gewichtsprozent.

Die neuen Verbindungen zeigen herbizide Wirkungen und eignen sich zur Beseitigung und Unterdrückung von unerwünschtem Pflanzenwuchs in Kulturpflanzen oder auf unbebautem Land. Dabei ist es selbstverständlich, daß einzelne Wirkstoffe unterschiedliche Intensitäten aufweisen oder in ihrer Wirkung gegenüber unerwünschten Pflanzen oder Kulturpflanzenarten differieren. Ihr Einfluß auf unerwünschte Pflanzen wird in den nachstehenden Tabellen erläutert. Die Versuchsserien wurden im Gewächshaus und im Freiland durchgeführt.

## I. Gewächshausversuche

Als Kulturgefäße dienten Plastikblumentöpfe mit 300 $cm^3$ Inhalt, die mit lehmigem Sand mit etwa 1,5% Humus gefüllt wurden. Die Samen der Testpflanzen entsprechend Tabelle 1 wurden nach Arten getrennt flach eingesät. Unmittelbar danach erfolgte bei Vorauflaufbehandlung das Aufbringen der Wirkstoffe auf die Erdoberfläche. Sie wurden hierbei in Wasser als Verteilungsmittel suspendiert oder emulgiert und mittels fein verteilender Düsen auf die Erde gespritzt. Nach dem Aufbringen der Mittel wurden die Töpfe leicht beregnet, um Keimung und Wachstum der Pflanzen anzuregen und gleichzeitig die chemischen Mittel zu aktivieren. Danach deckte man die Gefäße mit durchsichtigen Plastikhauben ab, bis die Pflanzen angewachsen waren. Diese Abdeckung bewirkte ein gleichmäßiges Keimen der Testpflanzen, sofern dies nicht durch die Chemikalien beeinträchtigt wurde, und verhinderte das Verdampfen leicht flüchtiger Wirkstoffe.

Zum Zwecke der Nachlaufaufbehandlung zog man die Pflanzen je nach Wuchsform in den Versuchsgefäßen erst bis zu einer Höhe von 3 bis 10 cm an und behandelte sie dann. Eine Abdeckung unterblieb. Die Aufstellung der Versuche erfolgt im Gewächshaus, wobei für wärmeliebende Arten wärmere Bereiche des Gewächshauses (25 bis 40°C) und für solche gemäßigter Klimate 15 bis 30°C bevorzugt wurden. Die Versuchsperiode erstreckte sich über 4 bis 6 Wochen. Während dieser Zeit wurden die Pflanzen gepflegt und ihre Reaktion auf die einzelnen Behandlungen wurde ausgewertet. Die folgenden Tabellen enthalten die Prüfsubstanzen, die jeweiligen Dosierungen in kg/ha Aktivsubstanz und die Testpflanzenarten. Bewertet wird nach einer Skala von 0 bis 100. Dabei bedeutet 0 keine Schädigung oder normaler Auflauf und 100 kein Aufgang der Pflanzen bzw. völlige Zerstörung zumindest der oberirdischen Sproßteile.

## II. Freilandversuche

Es handelt sich um Kleinparzellenversuche auf Standorten mit lehmigem Sand von pH 5–6 und 1 bis 1,5% Humusgehalt. Es werden Nachauflaufbehandlungen beschrieben, welche an jungen Testpflanzen in den frühen Wuchsstadien bis zu einer Höhe von 5 cm durchgeführt wurden. Die Unkrautflora verschiedenster Arten richtet sich nach der Jahreszeit (z. B. Frühjahrskeimer, Sommerkeimer). Die Substanzen werden in Wasser als Träger- und Verteilermedium emulgiert oder suspendiert und mit Hilfe einer motorgetriebenen, auf einen Geräteträger montierten Parzellenspitze ausgebracht. Bei Fehlen natürlicher Niederschläge wurde künstlich beregnet, um Keimung und Wachstum der Pflanzen zu gewährleisten. Alle Versuche dauerten mehrere Wochen. Danach wurde die Bewertung nach der Skala 0 bis 100 vorgenommen.

## Ergebnis

Die Tabellen 2 bis 13 enthalten die Ergebnisse der Versuche.

Es zeigte sich, daß die neuen 3-Aminopyrazolderivate interessante herbizide Eigenschaften bei Vor- und Nachauflaufanwendungen besitzen und sich dabei von den Vergleichsmitteln unterschneiden.

Fortsetzung

| Botanischer Name | Deutscher Name | Englischer Name |
|---|---|---|
| Citrus limon | Zitrone | lemon |
| Citrus maxima | Pampelmuse | grapefruits |
| Citrus reticulata | Mandarine | |
| Citrus sinensis | Apfelsine, Orange | orange trees |
| Coffea arabica (Coffea canephora, Coffea liberica) | Kaffee | coffee plants |
| Cucumis melo | Melone | melons |
| Cucumis sativus | Gurke | cucumber |
| Cynodon dactylon | Bermudagras | Bermudagrass in turfs and lawns |
| Daucus carota | Möhre | carrots |
| Elaeis guineensis | Ölpalme | oil palms |
| Fragaria vesca | Erdbeere | strawberries |
| Glycine max | Sojabohne | soybeans |
| Gossypium hirsutum (Gossypium arboreum, Gossypium herbaceum, Gossypium vitifolium) | Baumwolle | Cotton |
| Helianthus annuus | Sonnenblume | sunflowers |
| Helianthus tuberosus | Topinambur | |
| Hevea brasiliensis | Parakautschukbaum | rubber plants |
| Hordeum vulgare | Gerste | barley |
| Humulus lupulus | Hopfen | hop |
| Ipomoea batatas | Süßkartoffeln | sweet potato |
| Lactuca sativa | Kopfsalat | lettuce |
| Lens culinaris | Linse | lentils |
| Linum usitatissimum | Faserlein | flax |
| Lycopersicon lycopersicum | Tomate | tomato |
| Malus spp. | Apfel | apple trees |
| Manihot esculenta | Maniok | cassava |
| Medicago sativa | Luzerne | alfalfa (lucerne) |
| Mentha piperita | Pfefferminze | peppermint |
| Musa spp. | Obst- und Mehlbanane | banana plants |
| Nicotiana tabacum (N. rustica) | Tabak | tobacco |

Fortsetzung

| Botanischer Name | Deutscher Name | Englischer Name |
|---|---|---|
| Vaccinium vitis-idaea | Preißelbeere | cranberry |
| Vicia faba | Pferdebohnen | tick beans |
| Vigna sinensis (V. unguiculata) | Kuhbohne | cow peas |
| Vitis vinifera | Weinrebe | grapes |
| Zea mays | Mais | Indian corn, sweet corn, maize |

Zur weiteren Verbreiterung des Wirkungsspektrums der neuen Einzelsubstanzen, zur Erzielung synergistischer Effekte oder zum Verbessern der Dauerwirkung im Boden, lassen sich zahlreiche andere herbizide oder wachstumsregulierende Verbindungen als Mischungs- und Kombinationspartner heranziehen.

Außerdem ist es nützlich, die neuen erfindungsgemäßen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopatogenen Pilzen bzw. Bakterien. Von Interesse ist ferner die Mischbarkeit mit Mineralstofflösungen, welche zur Behebung von Ernährungs- oder Spurenelementmängeln eingesetzt werden.

Tabelle 1

Liste der Testpflanzen

| Botanischer Name | Deutsche Bezeichnung | Englische Bezeichnung |
|---|---|---|
| Abutilon theoprasti | Chinesischer Hanf | velvet leaf |
| Amaranthus retroflexus | Zurückgekrümmter Fuchsschwanz | redroot pigweed |
| Carthamus tinctorius | Färberdistel | safflower |
| Chenopodium album | Weißer Gänsefuß | lambsquarters (goosefoot) |
| Centaurea cyanus | Kornblume | cornflower |
| Cynodon dactylon | Hundszahngras | Bermudagrass |
| Cyperus difformis | – | smallflower umbrellaplant |
| Datura stramonium | Gemeiner Stechapfel | Jimsonweed |
| Daucus carota | Möhre | carrots |
| Echinochloa crus galli | Hühnerhirse | barnyardgrass |
| Euphorbia geniculata | Südamerik. Wolfsmilchart | Southamerican member of the spurge family |
| Galium aparine | Klettenlabkraut | catchweed bedstraw |
| Helianthus annuus | Sonnenblume | sunflower |
| Ipomoea spp. | Prunkwindearten | morningglory |
| Lamium purpureum | Rote Taubnessel | red deadnettle |
| Lolium multiflorum | Ital. Raygras | annual raygrass |

Tabelle 2

Herbizide Wirkung der Verbindungen gegen unerwünschte Pflanzen bei Vorauflaufanwendung im Gewächshaus

| Wirkstoff Nr. | kg/ha | Testpflanzen und % Schädigung Echinochloa crus galli | Euphorbia geniculata | Ipomoea spp. | Solanum nigrum | Sorghum halepense |
|---|---|---|---|---|---|---|
| 14 | 4,0 | 100 | 75 | 85 | 95 | 85 |
| 68 | 2,0 | 70 | 100 | 93 | – | 70 |
| 106 | 4,0 | 100 | 100 | 100 | 40 | 100 |
| 144 | 4,0 | 100 | 100 | 100 | 80 | 75 |
| 149 | 4,0 | 100 | 100 | 90 | 90 | 75 |
| 113 | 4,0 | 60 | – | 75 | 50 | 60 |
| | | Setaria spp. | | | | |
| 29 | 4,0 | 40 | – | 95 | 90 | 20 |
| 59 | 4,0 | 100 | – | 100 | 95 | 90 |
| 80 | 4,0 | 100 | – | 100 | 95 | 100 |
| 89 | 4,0 | 100 | – | 100 | 95 | 70 |
| 69 | 4,0 | 100 | – | 100 | 75 | 95 |

0 = ohne Schädigung, 100 = Pflanzen völlig zerstört.

Tabelle 4

Herbizide Wirkung bei Nachauflaufanwendung im Gewächshaus

| Wirk-stoff Nr. | kg/ha | Testpflanzen und % Schädigung Centaurea cyanus | Echinochloa crus galli | Galium aparine | Ipomoea spp. | Lolium multiflorum | Polygonum aviculare |
|---|---|---|---|---|---|---|---|
| 67 | 3,0 | 100 | 100 | 0 | 100 | 100 | 0 |
| 15 | 3,0 | 100 | 100 | 100 | 100 | 100 | 0 |
| 71 | 3,0 | 100 | 100 | 30 | 100 | 100 | 100 |
| 70 | 3,0 | 100 | 100 | 30 | 100 | 100 | 100 |
| 31 | 3,0 | 50 | 100 | 50 | 100 | 0 | 100 |
| 77 | 3,0 | 100 | – | 50 | 100 | 50 | 0 |
| 161 | 3,0 | 100 | 100 | 100 | 60 | 100 | 100 |
| 115 | 3,0 | 100 | 50 | 0 | 100 | 100 | 100 |
| 132 | 3,0 | 50 | 100 | 0 | 0 | 100 | – |
| 157 | 3,0 | 100 | 100 | 100 | 100 | 70 | 0 |
| 160 | 3,0 | 100 | 100 | 70 | 100 | 100 | 100 |
| 154 | 3,0 | 100 | 100 | 100 | 100 | 70 | 0 |
| 105 | 3,0 | 100 | 100 | 0 | 100 | 100 | 30 |
| 164 | 3,0 | 100 | 70 | 100 | 100 | 100 | 30 |
| 165 | 3.0 | 100 | 100 | 50 | 100 | 100 | 100 |
| 162 | 3,0 | 100 | 100 | 100 | 100 | 100 | 30 |
| 21 | 3,0 | 100 | 100 | 0 | 100 | 70 | 0 |
| 100 | 3,0 | 100 | 80 | 0 | 100 | 100 | – |
| 159 | 3,0 | 100 | 90 | 100 | 100 | 100 | 100 |
| 166 | 3,0 | 100 | 40 | 70 | 90 | 95 | – |
| 72 | 3,0 | 100 | 40 | 20 | 70 | 95 | – |
| 204 | 3,0 | 40 | 100 | 40 | 0 | 90 | – |
| 158 | 3,0 | 100 | 40 | 95 | 40 | 100 | – |
| 10 | 3,0 | 100 | 80 | 100 | 100 | 100 | – |
| 111 | 3,0 | 4,0 | 20 | 100 | 40 | 100 | – |
| 187 | 3,0 | 20 | 40 | 100 | 90 | 100 | – |
| 101 | 3,0 | 100 | 40 | 100 | 100 | 100 | – |
| 221 | 3,0 | 90 | 0 | 90 | 0 | 0 | – |
| 109 | 3,0 | 80 | 40 | 100 | 100 | 100 | – |

Tabelle 5

Herbizide Wirkung bei Nachauflaufbehandlung im Freiland

| Wirkstoff Nr. | kg/ha | Testpflanzen und % Schädigung<br>Chenopodium album | Matricaria spp. | Polygonum convolvulus | Polygonum persicaria | Raphanus raphanistrum/ Sinapis arvensis | Stellaria media |
|---|---|---|---|---|---|---|---|
| 14 | 2,0 | 80 | 20 | 30 | – | 70 | 70 |
| | 4,0 | 90 | 40 | 50 | – | 80 | 95 |
| 194 | 2,0 | 98 | 68 | 70 | 65 | 80 | 98 |
| | 4,0 | 100 | 88 | 95 | 95 | 98 | 100 |
| 68 | 2,0 | 100 | 90 | – | 75 | 85 | 100 |
| | 4,0 | 100 | 98 | – | 80 | 90 | 100 |
| 82 | 2,0 | 98 | 50 | 90 | 60 | 80 | 95 |
| | 4,0 | 100 | 95 | 100 | – | 100 | 100 |
| 106 | 2,0 | 100 | 65 | 90 | – | 90 | 85 |
| | 4,0 | 100 | 90 | 100 | – | 100 | 95 |
| 144 | 2,0 | 100 | 60 | 95 | – | 95 | 100 |
| | 4,0 | 100 | 90 | 100 | – | 100 | 100 |
| 149 | 2,0 | 100 | 60 | 100 | – | 100 | 100 |
| | 4,0 | 100 | 80 | 100 | – | 100 | 100 |
| 123 | 2,0 | 100 | 15 | – | 75 | – | 95 |
| | 4,0 | 100 | 20 | – | 90 | – | 100 |
| 30 | 2,0 | 100 | 20 | – | 80 | – | 100 |
| | 4,0 | 100 | 40 | – | 100 | – | 100 |
| B | | | | | | | |
| Bekannt | 2,0 | 100 | 100 | 80 | 40 | 100 | 100 |
| | 4,0 | 100 | 100 | 90 | 100 | 100 | 100 |
| Bekannt | 2,0 | 50 | 22 | 20 | 30 | – | – |
| | 4,0 | 88 | 57 | 95 | 62 | 100 | 83 |

C 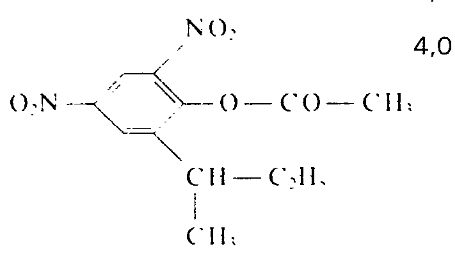

Tabelle 7

Selektive herbizide Wirkung in Erbsen bei Nachauflaufbehandlung im Gewächshaus

| Wirkstoff Nr. | kg/ha | Testpflanzen und % Schädigung Pisum sativum | Ipomoea spp. | Matricaria spp. | Setaria spp. | Sinapis alba | Stellaria media |
|---|---|---|---|---|---|---|---|
| 113 | 1,0 | 0 | 40 | 100 | 30 | – | – |
| | 2,0 | 10 | 75 | 100 | 100 | – | – |
| 30 | 1,0 | 0 | 60 | 0 | 40 | 100 | 100 |
| | 2,0 | 10 | 65 | 0 | 75 | 100 | 100 |
| 68 | 1,0 | 2 | 95 | 100 | 98 | 100 | 100 |
| | 2,0 | 4 | 99 | 100 | 98 | 100 | 100 |
| 14 | 1,0 | 12 | 90 | 7 | 98 | 90 | 95 |
| | 2,0 | 20 | 100 | 7 | 98 | 95 | 95 |
| 69 | 1,0 | 0 | 100 | 100 | 100 | 100 | 100 |
| | 2,0 | 0 | 100 | 100 | 100 | 100 | 100 |
| B | | | | | | | |
| Bekannt | 1,0 | 10 | 43 | 100 | 0 | 98 | 98 |
| | 2,0 | – | 55 | 100 | 30 | 98 | 98 |
| C | | | | | | | |
| Bekannt | 1,0 | 15 | 0 | 20 | – | 80 | 90 |
| | 2,0 | 28 | – | 20 | – | 100 | 100 |

0 = ohne Schädigung, 100 = Pflanzen vollkommen zerstört.

Tabelle 9

Selektive herbizide Wirkung bei Nachauflaufbehandlung im Gewächshaus

Grundkörper des Moleküls 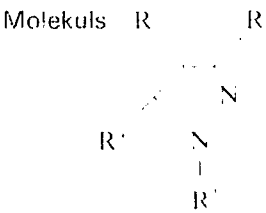

| Wirk-stoff Nr. | Substitutionen R[1] | R[2] | R[3] | R[4] | kg/ha | Testpflanzen und % Schädigung Daucus carota | Helian-thus annuus | Sorghum bicolor | Triticum aestivum | Zea mays | Echinochloa crus galli |
|---|---|---|---|---|---|---|---|---|---|---|---|
| 30 | | | | | 1,0 | 0 | 0 | 0 | 10 | 10 | 20 |
| | | | | | 2,0 | 0 | 10 | 10 | 23 | 10 | 27 |
| 41 | | | | | 1,0 | – | – | – | – | 10 | 10 |
| | | | | | 2,0 | – | – | – | 0 | 20 | 10 |
| 144 | | | | | 1,0 | – | 15 | – | 0 | 0 | 70 |
| | | | | | 2,0 | – | 20 | – | – | 0 | 100 |
| 149 | | | | | 1,0 | – | 10 | – | – | 0 | 100 |
| | | | | | 2,0 | – | 30 | – | – | 0 | 100 |
| Bekannt | $CH_3$ | $-CH_3$ | 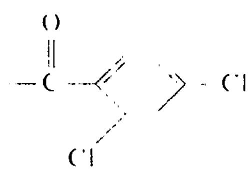 | 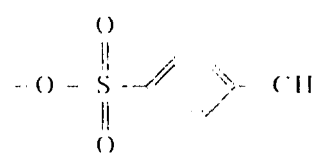 | 1,0 | – | – | – | 0 | – | 70 |
| D | | | | | 2,0 | – | – | – | 0 | – | 80 |

Fortsetzung

| Wirkstoff Nr. | Testpflanzen und % Schädigung | | | | | | | | |
|---|---|---|---|---|---|---|---|---|---|
| | Euphorbia geniculata | Ipomoea spp. | Lamium purpureum | Mercurialis annua | Polygonum persicaria | Sesbania exaltata | Setaria spp. | Sinapis alba | Stellaris media |
| 149 | 100 | 90 | 95 | 100 | – | 100 | 95 | 100 | – |
| | 100 | 100 | 95 | 100 | – | 100 | 95 | 100 | – |
| Bekannt | – | – | – | – | – | – | – | 0 | – |
| D | – | 20 | – | 30 | – | – | – | 25 | – |
| Bekannt | – | 0 | 0 | 0 | – | – | – | 15 | 60 |
| E | – | 0 | 0 | 0 | – | – | – | 45 | 60 |
| Bekannt | 100 | 13 | 0 | 10 | – | 40 | 0 | 0 | 30 |
| A | 100 | 15 | 0 | 50 | – | 50 | 20 | 0 | 45 |
| Bekannt | – | 0 | 100 | – | – | – | – | 80 | 90 |
| C | – | – | 100 | – | – | – | – | 100 | 100 |

0 = ohne Schädigung, 100 = Pflanzen vollkommen zerstört.

Tabelle 11

Herbizide Wirkung bei Erdnüssen und Baumwolle bei Nachauflaufanwendung im Gewächshaus

| Wirkstoff Nr. | kg/ha | Testpflanzen und % Schädigung Arachis hypogaea | Gossypium hirsutum | Alopecurus myosuroides | Amaranthus retroflexus | Chenopodium album | Echinochloa crus galli | Ipomoea spp. | Portulaca oleracea | Sesbania exaltata | Solanum nigrum |
|---|---|---|---|---|---|---|---|---|---|---|---|
| 67 | 1,0 | 10 | 0 | 90 | 100 | 100 | 60 | 100 | 100 | 100 | 100 |
| 68 | 0,5 | 10 | – | 90 | 100 | 100 | 70 | 75 | 100 | 95 | 100 |
| 69 | 0,5 | 10 | 0 | 95 | 50 | 100 | 40 | 75 | 100 | 60 | 100 |
| 70 | 1,0 | 0 | 0 | 95 | 50 | 90 | – | 60 | 100 | 60 | 100 |
| 198 | 1,0 | 20 | 20 | 95 | 50 | 100 | 95 | 90 | 100 | 100 | 100 |
| 77 | 2,0 | 0 | 10 | 95 | – | 90 | 100 | 60 | 100 | 95 | 100 |
| 21 | 1,0 | 0 | 0 | 60 | – | 100 | – | 100 | – | – | – |
| 165 | 0,5 | 15 | 0 | 85 | 100 | 100 | – | – | 100 | – | 40 |
| 106 | 0,5 | 10 | 15 | 90 | 100 | 90 | – | – | 70 | – | – |
| 153 | 2,0 | 0 | 20 | – | 100 | 100 | 40 | – | 100 | – | 100 |
| 154 | 0,5 | 5 | 20 | 80 | 100 | 100 | 70 | 80 | 100 | 55 | 100 |
| 31 | 2,0 | 0 | 0 | – | 100 | 90 | – | 100 | 100 | 70 | – |
| 160 | 1,0 | 0 | 0 | 95 | 100 | 100 | 100 | – | 100 | 100 | 100 |
| 80 | 1,0 | 5 | 5 | – | – | – | 100 | 50 | 100 | 100 | – |
| 161 | 0,5 | 15 | 0 | 100 | – | 100 | 90 | – | 100 | 100 | 100 |

0 ohne Schädigung, 100 = Pflanzen völlig zerstört.

Beispiel 11

Man vermischt 90 Gewichtsteile der Verbindung 1 mit 10 Gewichtsteilen N-Methyl-α-pyrrolidon und erhält eine Lösung, die zur Anwendung in Form kleinster Tropfen geeignet ist.

Beispiel 12

20 Gewichtsteile der Verbindung 2 werden in einer Mischung gelöst, die aus 80 Gewichtsteilen Xylol, 10 Gewichtsteilen des Anlagerungsproduktes von 8 bis 10 Mol Äthylenoxid an 1 Mol Ölsäure-N-monoäthanolamid, 5 Gewichtsteilen Calciumsalz der Dodecylbenzolsulfonsäure und 5 Gewichtsteilen des Anlagerungsproduktes von 40 Mol Äthylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

Beispiel 13

20 Gewichtsteile der Verbindung 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel 14

20 Gewichtsteile der Verbindung 1 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel 15

20 Gewichtsteile des Wirkstoffs 2 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

Beispiel 16

3 Gewichtsteile der Verbindung 3 werden mit 97 Gewichtsteilen feinteiligem Kaolin innig vermischt. Man erhält auf diese Weise ein Stäubemittel, das 3 Gewichtsprozent des Wirkstoffs enthält.

Beispiel 17

30 Gewichtsteile der Verbindung 4 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel 18

40 Gewichtsteile des Wirkstoffs 1 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,4 Gewichtsprozent Wirkstoff enthält.

$R^4$ denotes $-COR^6$, $R^6$ denoting methoxy or isopropoxy or alkyl, and
$R^5$ denotes hydrogen or methyl,
and the salts thereof.

2. A herbicide characterized in that it contains a substituted 3-aminopyrazole as claimed in claim 1.

3. A process for controlling the growth of unwanted plants, characterized in that the soil or the plants are treated with a substituted 3-aminopyrazole as claimed in claim 1.

4. 1-Acetyl-3-anilino-4-methoxycarbonyl-5-methylpyrazole.

5. 1-Propionyl-3-anilino-4-methoxycarbonyl-5-methylpyrazole.

6. 3-sec-Butylamino-4-methoxycarbonyl-5-methylpyrazole.

7. 3-(3-Methoxyanilino)-4-methoxycarbonyl-5-methylpyrazole.

8. 1-Phenoxycarbonyl-3-anilino-4-methoxycarbonyl-5-methylpyrazole.

9. 1-Acetyl-3-(4-fluoroanilino)-4-methoxycarbonyl-5-methylpyrazole.

**Revendications**

1. 3-aminopyrazoles de formule

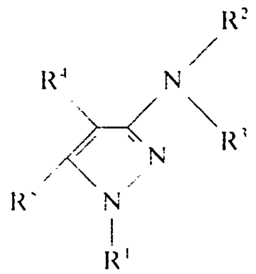

ou

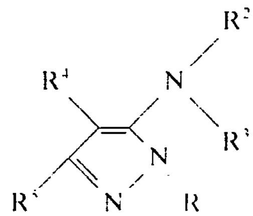

dans laquelle
$R^1$ représente hydrogène, un reste alcoxycarbonyle, ou dialkylaminocarbonyle, phénoxycarbonyle ou acyle, éventuellement substitué par du chlore,
$R^2$ l'hydrogène ou un reste alkyle,
$R^3$ un reste cycloalkyle, alcényle, ou phényle, éventuellement substitué par un alkyle, alcoxy, halogénoalkyle, halogénoalcoxy ou un halogène, ou un reste alkyle, éventuellement substitué par un alcoxy ou un phényle,
$R^2$ et $R^3$ ensemble un reste polyméthylène éventuellement interrompu par un atome d'oxygène,
$R^4$ le reste $-COR^6$, où $R^6$ est un reste méthoxy ou isopropoxy ou un reste alkyle,
$R^5$ hydrogène ou méthyle
et les sels de ces aminopyrazoles.

2. Herbicide caractérisé par le fait qu'il contient un 3-aminopyrazole substitué selon la revendication 1.

3. Procédé de lutte contre la croissance de plantes indésirables, caractérisé par le fait que l'on traite le sol ou les plantes avec un 3-aminopyrazole substitué selon la revendication 1.

4. 1-acétyl-3-anilino-4-méthoxycarbonyl-5-méthylpyrazole.

5. 1-propionyl-3-anilino-4-méthoxycarbonyl-5-méthylpyrazole.

6. 3-sek.-butylamino-4-méthoxycarbonyl-5-méthylpyrazole.

7. 3-(3-méthoxyanilino)-4-méthoxycarbonyl-5-méthylpyrazole.

8. 1-phénoxycarbonyl-3-anilino-4-méthoxycarbonyl-5-méthylpyrazole.

9. 1-acétyl-3-(4-fluoranilino)-4-méthoxycarbonyl-5-méthylpyrazole.